# EUROPEAN PATENT APPLICATION

(11) **EP 1 498 410 A1**
(43) Date of publication of application: **19.01.2005**
(21) Application number: 03707171.9
(22) Date of filing: 28.02.2003
(51) Int. Cl.: C07C 229/08, C07C 227/42

(54) **PROCESS FOR PRODUCING TERT-LEUCINE**

(30) Priority: 06.03.2002 JP 2002061082
(71) Applicant: Ajinomoto Co., Inc., Tokyo 104-8315 (JP)
(72) Inventor: NAGASHIMA, Kazutaka, Yokkaichi-shi, Mie 510-0885 (JP); TAKEMOTO, Tadashi, Kawasaki-shi, Kanagawa 210-8681 (JP)
(74) Representative: Nicholls, Kathryn Margaret
(86) International application number: PCT/JP2003/002310
(87) International publication number: WO 2003/074470

(57) **Abstract**

The present invention provides a method of producing tert-leucine, which includes reacting tert-leucine with substituted benzenesulfonic acid represented by the following formula (1) 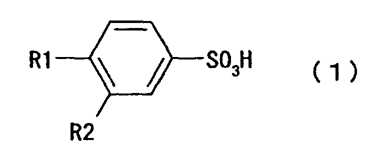 wherein R1 and R2 are each independently a hydrogen atom, an alkyl group or a halogen atom, except a compound wherein R1 and R2 are both hydrogen atoms, in a solvent, to give tert-leucine·substituted benzenesulfonate represented by the following formula (2) 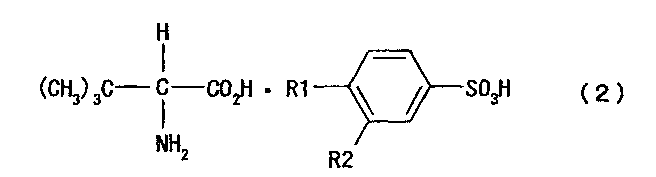 wherein R1 and R2 are the same substituents as above, separating the salt by crystallization and then dissociating the salt. According to the present invention, a production method of tert-leucine is provided, which includes forming a tert-leucine salt hardly soluble in a solvent and crystallization thereof to efficiently recover tert-leucine from the solvent.

## Description

### Technical Field

The present invention relates to a production method of tert-leucine and novel tert-leucine·substituted benzenesulfonate obtained as an intermediate in the production method.

### Background Art

tert-Leucine is a nonprotein amino acid, and an optically active form thereof is particularly drawing attention as a synthesis starting material of various peptide pharmaceutical products, as well as utilized for asymmetric synthesis of amino acid derivative.

As a production method of an optically active tert-leucine, a method comprising reacting a reagent for optical resolution with racemic tert-leucine obtained by chemical synthesis to allow optical resolution of the produced diastereomer salt has been considered from various aspects (e.g., (1) T. Tanabe et al., Bulletin the Chemical Society of Japan, 41, 2178 (1968)), (2) T. Miyazawa et al., ibid, 52, 1539 (1979)), (3) D.A. Jaeger et al., Journal of American Chemical Society, 101, 717 (1979), (4) JP-B-63-54700, (5) JP-A-5-271169 and the like).

However, these methods are not necessarily satisfactory from the aspect of production cost, because an expensive reagent for optical resolution, which has high optical purity, needs to be prepared separately and used.

On the other hand, as a method that does not require such expensive reagent for optical resolution, a method comprising applying an enzyme reaction to directly produce an optically active tert-leucine or a precursor thereof has been considered. For example, (6) a method of producing an optically active tert-leucine, which comprises aminating 2-oxo-3,3-dimethyl-butanoic acid by an enzyme method (G. Krix et al., Journal of Biotechnology, 53, 29 (1997), JP-B-3182397, US6197558B1), (7) a method of producing L-tert-leucine, which comprises converting DL-5-tert-butyl hydantoin to N-carbamoyl-L-tert-leucine by an enzyme method, and removing a carbamoyl group (JP-A-7-222593) and the like have been reported.

In the case of a method using an enzyme, the reaction is generally carried out in an aqueous solution, but the above-mentioned references do not consider at all how to efficiently recover tert-leucine in a reaction solution. As an industrial method to separate amino acid from a reaction solution containing various contaminants, a method for separating crystals by crystallization is first considered. However, since tert-leucine has high solubility in aqueous solutions, efficient recovery by crystallization is difficult (e.g., solubility of L-tert-leucine in water is 12.7 wt% (10°C), solubility in 1N aqueous hydrochloric acid solution is 20.4 wt% (10°C)). As mentioned above, a method for efficiently recovering tert-leucine from a solvent, particularly an aqueous solution, has been desired.

### Disclosure of the Invention

The present invention aims at providing a method for efficiently recovering tert-leucine dissolved in a solvent, particularly water and the like that are good solvents for tert-leucine, and specifically aims at providing a production method of tert-leucine, which comprises forming a tert-leucine salt that is hardly soluble in the above solvents, crystallization thereof, thereby efficiently recovering tert-leucine from the solvent, and a dissociation treatment of the salt.

The present inventors have conducted intensive studies in an attempt to solve the above-mentioned problems, and found that tert-leucine can be efficiently recovered from a solvent by crystallization of tert-leucine dissolved in a solvent as particular substituted benzenesulfonate, which resulted in the completion of the present invention. Accordingly, the present invention includes the following.
[1] a method of producing tert-leucine, which comprises reacting tert-leucine with substituted benzenesulfonic acid represented by the following formula (1) 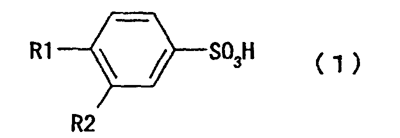 wherein R1 and R2 are each independently a hydrogen atom, an alkyl group or a halogen atom, except a compound wherein R1 and R2 are both hydrogen atoms, in a solvent, to give tert-leucine·substituted benzenesulfonate represented by the following formula (2) 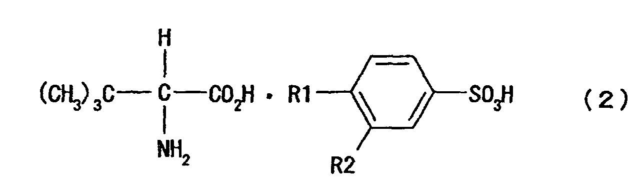 wherein R1 and R2 are the same substituents as above, separating the salt by crystallization and then dissociating the salt.
[2] The production method of the above-mentioned [1], wherein the solvent is water.
[3] The production method of the above-mentioned [2], wherein the aqueous solvent has pH 0.1-3.0 during crystallization.
[4] The production method of any of the above-mentioned [1] to [3], wherein the tert-leucine is in an optically active form.
[5] A tert-leucine·substituted benzenesulfonate represented by the following formula (2): 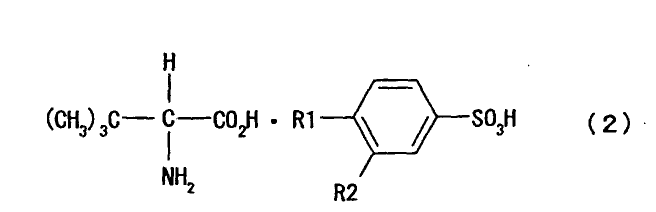
wherein R1 and R2 are each independently a hydrogen atom, an alkyl group or a halogen atom, except a compound wherein R1 and R2 are both hydrogen atoms.

### Brief Description of Drawings

Fig. 1 shows a powder X-ray diffraction pattern of L-tert-leucine·3,4-dimethylbenzenesulfonate.
Fig. 2 shows a powder X-ray diffraction pattern of D-tert-leucine·3,4-dimethylbenzenesulfonate.
Fig. 3 shows a powder X-ray diffraction pattern of D,L-tert-leucine·3,4-dimethylbenzenesulfonate.
Fig. 4 shows a powder X-ray diffraction pattern of L-tert-leucine·p-toluenesulfonate.
Fig. 5 shows a powder X-ray diffraction pattern of L-tert-leucine·m-toluenesulfonate.
Fig. 6 shows a powder X-ray diffraction pattern of L-tert-leucine·p-ethylbenzenesulfonate.
Fig. 7 shows a powder X-ray diffraction pattern of L-tert-leucine·p-chlorobenzenesulfonate.

### Detailed Description of the Invention

The present invention is explained in detail in the following.

In the substituted benzenesulfonic acid represented by the formula (1) to be used in the present invention, R1 and R2 are each independently a hydrogen atom, an alkyl group or a halogen atom but a compound wherein R1 and R2 are both hydrogen atoms is excluded. As the alkyl group, alkyl group having 1 to 4 carbon atoms, such as methyl group, ethyl group, n-propyl group, isopropyl group, n-butyl group, isobutyl group, tert-butyl group can be preferably mentioned, and more preferably, methyl group, ethyl group, n-propyl group and isopropyl group can be mentioned. As the halogen atom, fluorine atom, chlorine atom and bromine atom can be mentioned.

Particularly preferable examples of substituted benzenesulfonic acid (1) include a compound wherein at least one of R1 and R2 is methyl group, ethyl group or chlorine atom can be mentioned. Specifically, 3,4-dimethylbenzenesulfonic acid, p-toluenesulfonic acid, m-toluenesulfonic acid, p-ethylbenzenesulfonic acid, p-chlorobenzenesulfonic acid and the like can be mentioned, and preferably, 3,4-dimethylbenzenesulfonic acid and the like can be mentioned.

A compound wherein R1 and R2 are both hydrogen atoms and a compound wherein the substituted positions of R1 and R2 are different are difficult to afford crystals of tert-leucine· substituted benzenesulfonate (2).

The substituted benzenesulfonic acid (1) can be industrially easily prepared by sulfonation of various kinds of substituted benzene. In the present invention, moreover, it is not always necessary to use purified substituted benzenesulfonic acid (1), and a crude product, such as a reaction mixture, wherein substituted benzene is sulfonated, can be also used. In addition, as the form of use, use as a free acid, or use in the form of a salt soluble in the solvent to be used in the present invention, such as alkali metal salts (e.g., sodium salt, potassium salt and the like), alkaline earth metal salts (e.g., calcium salt and the like), salts with organic base (e.g., ammonium salt and the like), and the like is acceptable.

The tert-leucine to be applied to the method of the present invention may be in an optically active form (e.g., L-tert-leucine or D-tert-leucine), or a racemate, or a mixture of these at any amount ratio.

When an optically active form is used as a starting material tert-leucine in the present invention, the tert-leucine·substituted benzenesulfonate (2) and tert-leucine obtained by the production method of the present invention are in an optically active form, like the starting material. When a racemate is used as a starting material, moreover, tert-leucine·substituted benzenesulfonate (2) and tert-leucine are obtained as racemates. When tert-leucine to be used as a starting material comprises an object optically active form and a different optically active form in a part thereof, the optically active form contained in the starting material in a greater amount can be obtained as an object product. Therefore, it is possible to obtain tert-leucine·substituted benzenesulfonate (2), and tert-leucine, which have high optical purity. When the method of the present invention is used to increase the optical purity, the content of the object optically active form in the starting material tert-leucine is preferably not less than 60 wt%, more preferably not less than 70 wt%, most preferably not less than 75 wt%, of the entire tert-leucine.

The solvent to be used in the present invention is not particularly limited as long as tert-leucine·substituted benzenesulfonate (2) is hardly soluble therein. The method of the present invention is particularly preferably applied to a solvent that is a good solvent to tert-leucine but a poor solvent to tert-leucine·substituted benzenesulfonate (2). As such solvent, for example, water, a mixed solvent of water and a solvent miscible with water, such as methanol, ethanol and the like, and the like can be mentioned.

According to the production method of the present invention, tert-leucine and substituted benzenesulfonic acid (1) are first reacted in a suitable solvent to give tert-leucine·substituted benzenesulfonate, and this salt is separated by crystallization.

In the salt forming reaction, the order of addition is not particularly limited, and, for example, substituted benzenesulfonic acid (1) (or a solution thereof) may be added to tert-leucine dissolved in a solvent; tert-leucine and substituted benzenesulfonic acid (1) (or a solution thereof) may be simultaneously or successively added to a solvent; tert-leucine (preferably a solution thereof) may be added to substituted benzenesulfonic acid (1) dissolved in a solvent and the like.

The amount of substituted benzenesulfonic acid (1) to be used in the present invention is not particularly limited, and it is used within the range of generally 0.5- to 2.0-fold molar amount, preferably 0.9- to 1.2-fold molar amount, relative to tert-leucine.

The method of crystallizing the formed salt is not particularly limited and, for example, a method comprising heating tert-leucine and substituted benzenesulfonic acid (1) as necessary for dissolution, performing cooling crystallization under static or stirring condition, a method comprising crystallization by concentration under suitable temperature conditions, and the like can be mentioned.

When crystallization by concentration is to be performed and substituted benzenesulfonic acid (1) is to be added to tert-leucine dissolved in a solvent, the substituted benzenesulfonic acid (1) may be added before and after concentration and in any stage during concentration.

The temperature during crystallization may be from a freezing point to a boiling point of the solvent to be used. When water is used as a solvent, for example, preferable temperature range is from 5°C to 80°C. The concentration of tert-leucine·substituted benzenesulfonate (2) during crystallization is not particularly limited, but it is preferably 5-70 wt% of the solvent.

When water is used as a solvent, the pH during crystallization is preferably set to not more than 3 for efficient crystallization of tert-leucine·substituted benzenesulfonate (2). More preferable pH range is pH 0.1-3.0, and pH 0.1-2.5 is more preferable and pH 0.5-2.0 is particularly preferable. When pH is to be adjusted, pH adjustment may be performed any time of before and after salt formation, during crystallization and the like. The acid to be used for pH adjustment is not particularly limited, and, for example, inorganic acids such as hydrochloric acid, sulfuric acid and the like are preferably used.

Where necessary, a seed crystal may be added during crystallization. For example, when tert-leucine to be used as a starting material contains an object optically active form as well as a different optically active form in a part thereof, a seed crystal of the same kind as the desired optically active form can be added to facilitate precipitation of the object optically active form, to increase the optical purity and the like.

The crystals of tert-leucine substituted benzenesulfonate (2) precipitated in the crystallization step can be easily separated by a conventional solid-liquid separation method such as filtration, centrifugal separation and the like. In addition, the separated crystals may be further purified, as necessary, by a step of washing, dehydration, recrystallization and the like.

The tert-leucine can be obtained by a dissociation treatment of the thus-obtained crystals of tert-leucine· substituted benzenesulfonate (2). The dissociation treatment can be performed according to a method known to those of ordinary skill in the art. For example, a method comprising dissolving or suspending the salt in a single solvent of water or alcohols or a mixed solvent of these, and neutralizing the mixture with a base such as sodium hydroxide, ammonia, triethylamine, pyridine and the like, a method comprising passing an aqueous solution of the salt through a column packed with a weak basic anion exchange resin, or passing the aqueous solution through a column packed with a strong acidic cation exchange resin, eluting the solution with ammonia, and evaporating the obtained transfluent solution or eluate under reduced pressure or crystallization thereof and the like can be mentioned.

The obtained tert-leucine can be further purified as necessary by recrystallization with a suitable solvent such as alcohol and the like.

Since substituted benzenesulfonic acid (1) is a chemically stable compound, it can be re-used by recovering from a mother liquor after dissociation of the salt, or eluate or transfluent solution from the resin treatment.

The production method of the present invention can be particularly preferably used for, for example, a system using water as a solvent, such as production of tert-leucine using an enzyme and the like. In this case, substituted benzenesulfonic acid (1) is added to a reaction solution containing tert-leucine, which is obtained by an enzyme method, or a reaction solution containing tert-leucine, which is obtained by producing a precursor of tert-leucine by an enzyme method and converting the precursor to tert-leucine, crystallization can be performed by the aforementioned crystallization method to give crystals of tert-leucine· substituted benzenesulfonate (2). Since contaminants in the enzyme reaction solution and the like can be efficiently removed during the above steps, the method is extremely useful as a method for selectively separating or purifying tert-leucine.

### Examples

The present invention is explained in more detail in the following by referring to Examples, which are not to be construed as limitative.

### <HPLC analysis condition 1>

[column] inertsil ODS-2 (4.6φ×250 mm)
[column bath temperature] 40°C
[mobile phase] liquid A: 0.1 mol/1 potassium dihydrogen phosphate (pH 2.8) - acetonitrile (95:5), liquid B: 0.1 mol/l potassium dihydrogen phosphate (pH 2.8)- acetonitrile (50:50)
[gradient program]:

| Analysis time (minute) | Liquid A (%) | Liquid B (%) |
|---|---|---|
| 0 | 100 | 0 |
| 20 | 70 | 30 |
| 30 | 50 | 50 |
| 35 | 0 | 100 |
| 45 | 0 | 100 |

[flow rate] 1.0 ml/min
[detection] UV 210 nm

### <HPLC analysis condition 2>

[column] SUMICHIRAL OA-5000 (4.6φ×150 mm)
[column bath temperature] 30°C
[mobile phase] 2 mmol/l aqueous copper sulfate solution-methanol (90:10)
[flow rate] 1.0 ml/min
[detection] UV 254 nm

### <Example 1>

### Crystallization of L-tert-leucine·3,4-dimethylbenzenesulfonate - (1)

L-tert-leucine (1.31 g, 0.010 mol) was dissolved in 1N aqueous hydrochloric acid solution (10 ml) at 40°C. To the obtained solution was added aqueous 3,4-dimethylbenzenesulfonic acid solution (4.01 g, 0.011 mol) having a concentration of 51.9 wt% (also containing free sulfuric acid 4.4 wt%, 2,3-dimethylbenzenesulfonic acid 4.7 wt% relative to 3,4-dimethylbenzenesulfonic acid weight, dixylyl sulfone 0.19 wt% relative to 3,4-dimethylbenzenesulfonic acid weight and xylene 0.06 wt% relative to 3,4-dimethylbenzenesulfonic acid weight) at the same temperature and crystals were allowed to precipitate overnight at 10°C. The precipitated crystals were collected by filtration, and vacuum dried at 55°C for 2 hr to give L-tert-leucine·3,4-dimethylbenzenesulfonate (2.87 g). The crystals were analyzed by HPLC analysis condition 1. As a result, L-tert-leucine content was 41.0 wt% and the rate of recovery was 90.0%.

### <Example 2>

### Crystallization of L-tert-leucine·3,4-dimethylbenzenesulfonate - (2)

L-tert-leucine (1.31 g, 0.010 mol) and sodium 3,4-dimethylbenzenesulfonate (2.08 g, 0.010 mol) were dissolved in 1N aqueous hydrochloric acid solution (10 ml) at 80°C. Crystals were allowed to precipitate overnight at 10°C. The precipitated crystals were collected by filtration, and vacuum dried at 55°C for 14 hr to give L-tert-leucine·3,4-dimethylbenzenesulfonate (2.70 g). The crystals were analyzed by HPLC analysis condition 1. As a result, L-tert-leucine content was 41.2 wt% and the rate of recovery was 84.9%.

### <Comparative Example 1>

### Crystallization of L-tert-leucine·2,4-dimethylbenzenesulfonate

L-tert-leucine (1.31 g, 0.010 mol) was dissolved in 1N aqueous hydrochloric acid solution (10 ml) at 40°C. Sodium 2,4-dimethylbenzenesulfonate (2.08 g, 0.010 mol) was added to the obtained solution at the same temperature, and crystals were allowed to precipitate overnight at 10°C. However, precipitation of crystals was not confirmed.

### <Comparative Example 2>

### Crystallization of L-tert-leucine·benzenesulfonate

L-tert-leucine (1.31 g, 0.010 mol) and benzenesulfonic acid monohydrate (1.76 g, 0.010 mol) were dissolved in 1N aqueous hydrochloric acid solution (10 ml) at 55°C and crystals were allowed to precipitate overnight at 10°C. However, precipitation of crystals was not confirmed.

### <Comparative Example 3>

### Crystallization of L-tert-leucine

L-tert-leucine (1.31 g, 0.010 mol) was dissolved in 1N aqueous hydrochloric acid solution (10 ml) at 40°C and crystals were allowed to precipitate overnight at 10°C. However, precipitation of crystals was not confirmed.

### <Reference Example 1>

### Decarbamoylation reaction of N-carbamoyl-L-tert-leucine

N-Carbamoyl-L-tert-leucine (15.0 g, 0.086 mol) and pure water (250 ml) were charged in a 500 ml four-necked vessel equipped with a gas outlet, a stirrer, a temperature indicator and an introduction tube reaching the lower part of the reaction vessel, and this slurry was heated to 40°C and stirred for a while. Thereto was added 35 wt% hydrochloric acid (34.7 g) with stirring, and aqueous sodium nitrite solution (31.3 g) containing sodium nitrite (6.26 g) was added from the introduction tube over 45 min using a constant rate pump. After the completion of the addition, the mixture was reacted at the same temperature for 11 hr with stirring. By this reaction, 328 g of the reaction mixture was obtained. The reaction mixture was analyzed under HPLC analysis condition 1. As a result, the content of L-tert-leucine was 3.12 wt% and the yield was 90.6%.

### <Example 3>

### Crystallization of L-tert-leucine·3,4-dimethylbenzenesulfonate and production of L-tert-leucine - (1)

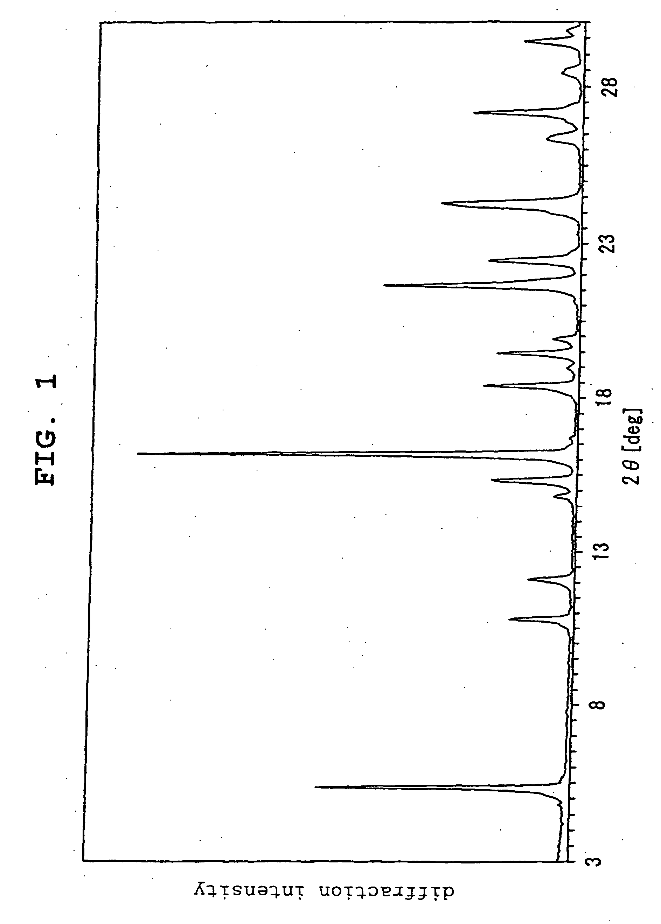

The reaction mixture obtained in Reference Example 1 (322 g, containing L-tert-leucine 0.077 mol) was concentrated under reduced pressure to give 80.0 g of a concentrated solution. Thereto was added the same aqueous 3,4-dimethylbenzenesulfonic acid solution (30.4 g) (containing 0.085 mol of 3,4-dimethylbenzenesulfonic acid) as used in Example 1 and treated at 90°C to give a homogeneous solution. This solution was allowed to cool to 70°C and then cooled from 70°C to 10°C over 12 hr, and crystals were allowed to precipitate at 10°C for 13 hr. The precipitated crystals were collected by filtration, washed with cold water (4 ml) and acetonitrile (30 ml), and vacuum dried at 40°C for 2 hr to give L-tert-leucine·3,4-dimethylbenzenesulfonate (20.6 g). The crystals were analyzed under HPLC analysis condition 1. As a result, L-tert-leucine content was 41.0 wt% and the rate of recovery was 83.9%. In addition, by the analysis under HPLC analysis condition 2, the optical purity was 100%ee. Furthermore, measurement by powder X-ray diffraction method (using CuKα ray as a radiation source) revealed a powder X-ray diffraction pattern as shown in Fig. 1. The melting point was 230-235°C (decomposition).

The obtained salt (5.84 g, containing 0.018 mol of L-tert-leucine) was dissolved in pure water (160 ml) at 30°C, passed through a column packed with an ion exchange resin (DIAION SK1B, product name, MITSUBISHI CHEMICAL CORPORATION, H⁺ type, 30 ml) at a space velocity (SV) 2, and washed with pure water (60 ml) to recover 3,4-dimethylbenzenesulfonic acid in a transfluent solution and a washing solution. An analysis under HPLC analysis condition 1 revealed the rate of recovery of 3,4-dimethylbenzenesulfonic acid was quantitative. Elution with 5 wt% aqueous ammonia (90 ml) and washing with pure water (90 ml) gave L-tert-leucine in the eluate and washing solution. The eluate and washing solution were combined to recover L-tert-leucine. An eluted fraction (179 g) was obtained, which was analyzed under HPLC analysis condition 1 to find that an L-tert-leucine content was 1.33 wt% and the rate of recovery of L-tert-leucine was quantitative. Subsequently, the eluted fraction was concentrated under reduced pressure to 6.99 g at 50°C, and isopropyl alcohol (70 ml) was added. The mixture was heated to 70°C, stirred for 10 min, cooled to 10°C and crystals were allowed to precipitate for 3 hr. The precipitated crystals were collected by filtration, and vacuum dried at 60°C for 4 hr to give L-tert-leucine (2.32 g). The crystals were analyzed under HPLC analysis condition 1 to find that the L-tert-leucine content was 99.3 wt% and the rate of recovery relative to the salt was 96.5%. In addition, the analysis under HPLC analysis condition 2 revealed an optical purity of 100%ee.

Separately, the obtained salt (9.52 g, containing 0.030 mol of L-tert-leucine) was dissolved in pure water (200 ml) at 45°C, passed through a column packed with an ion exchange resin (DIAION WA30, product name, MITSUBISHI CHEMICAL CORPORATION, OH⁻ type, 30 ml) at a space velocity (SV) 2, and washed with pure water (60 ml). The transfluent solution and a washing solution are combined and L-tert-leucine was recovered. As a result, a transfluent fraction (274 g) was obtained, an L-tert-leucine content was 1.42 wt% and the rate of recovery of L-tert-leucine was quantitative. Subsequently, a transfluent fraction was concentrated under reduced pressure to 11.9 g at 50°C and isopropyl alcohol (90 ml) was added. The mixture was heated to 70°C, stirred for 10 min, cooled to 10°C and crystals were allowed to precipitate for 17 hr. The precipitated crystals were collected by filtration, and vacuum dried at 60°C for 4 hr to give L-tert-leucine (3.79 g). The crystals were analyzed under HPLC analysis condition 1 to find that the L-tert-leucine content was 99.9 wt% and the rate of recovery relative to the salt was 97.0%. In addition, the analysis under HPLC analysis condition 2 revealed an optical purity of 100%ee. Elution with 5 wt% aqueous ammonia (90 ml) and washing with pure water (90 ml) gave 3,4-dimethylbenzenesulfonic acid in the eluate and washing solution. An analysis under HPLC analysis condition 1 revealed that the rate of recovery of 3,4-dimethylbenzenesulfonic acid was quantitative.

### <Example 4>

### Crystallization of L-tert-leucine·3,4-dimethylbenzenesulfonate and production of L-tert-leucine - (2)

To pure water (45 ml) were added L-tert-leucine (3.00 g, 0.023 mol), D-tert-leucine (0.94 g, 0.007 mol) and the same aqueous 3,4-dimethylbenzenesulfonic acid solution (8.22 g, containing 0.023 mol of 3,4-dimethylbenzenesulfonic acid) as used in Example 1 and the mixture was treated at 80°C to give a homogeneous solution. This solution was allowed to cool to 60°C and L-tert-leucine·3,4-dimethylbenzenesulfonate (0.218 g) obtained in Example 2 was added as a seed crystal. The mixture was cooled from 60°C to 10°C over 10 hr and the crystals were allowed to precipitate at 10°C for 15 hr. The precipitated crystals were collected by filtration, washed with cold water (4 ml) and acetonitrile (20 ml) and vacuum dried at 40°C for 3 hr to give tert-leucine·3,4-dimethylbenzenesulfonate (4.84 g). The crystals were analyzed under HPLC analysis condition 1 to find that the tert-leucine content was 41.3 wt%, and the rate of recovery to the L-tert-leucine used was 64.7%. In addition, analysis under HPLC analysis condition 2 revealed that the optical purity was 96.7%ee. Furthermore, to the obtained crystal (4.50 g) was added pure water (10 ml), the mixture was dissolved at 90°C, and the crystals were allowed to precipitate overnight at 10°C. The precipitated crystals were collected by filtration, washed with cold water (2 ml) and acetonitrile (20 ml) and vacuum dried at 60°C for 3 hr to give L-tert-leucine·3,4-dimethylbenzenesulfonate (3.83 g). The crystals were analyzed under HPLC analysis condition 1 to find that the L-tert-leucine content was 41.3 wt% and the rate of recovery was 85.0%. In addition, the analysis under HPLC analysis condition 2 revealed an optical purity of 100%ee. Measurement by powder X-ray diffraction method (using CuKα ray as a radiation source) revealed a powder X-ray diffraction pattern similar to that shown in Fig. 1. The melting point was 229-234°C (decomposition).

The obtained salt (3.81 g, containing 0.012 mol of L-tert-leucine) was dissolved in pure water (80 ml) at 45°C, and treated in the same manner as in Example 3 using an ion exchange resin (DIAION WA30, product name, MITSUBISHI CHEMICAL CORPORATION, OH- type, 30 ml) to recover L-tert-leucine. As a result, a transfluent fraction (151 g) was obtained, an L-tert-leucine content was 1.00 wt% and the rate of recovery of L-tert-leucine relative to the salt was 95.8%. Subsequently, a transfluent fraction was concentrated under reduced pressure to 9.33 g at 50°C and isopropyl alcohol (72 ml) was added. The mixture was heated to 70°C, stirred for 30 min, cooled to 10°C and crystals were allowed to precipitate for 4 hr. The precipitated crystals were collected by filtration, and vacuum dried at 60°C for 4 hr to give L-tert-leucine (1.33 g). The crystals were analyzed under HPLC analysis condition 1 to find that the L-tert-leucine content was 99.4 wt% and the rate of recovery was 87.9%. In addition, the analysis under HPLC analysis condition 2 revealed an optical purity of 100%ee.

### <Example 5>

### Crystallization of D-tert-leucine·3,4-dimethylbenzenesulfonate and production of D-tert-leucine

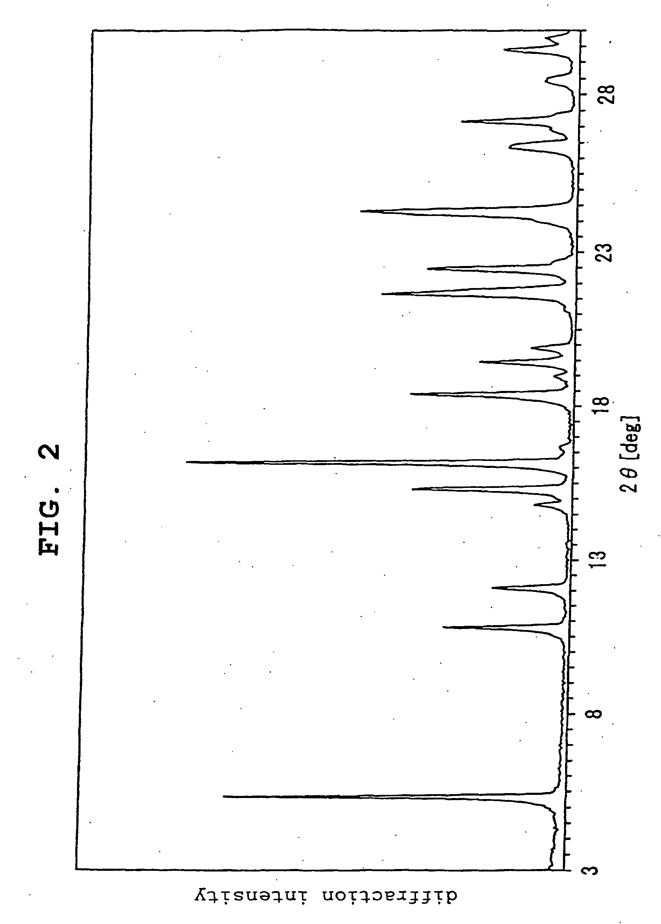

To 1N aqueous hydrochloric acid solution (10 ml) were added D-tert-leucine (1.31 g, 0.010 mol) and the same aqueous 3,4-dimethylbenzenesulfonic acid solution (3.58 g, containing 0.010 mol of 3,4-dimethylbenzenesulfonic acid) as used in Example 1, and the mixture was treated at 80°C to give a homogeneous solution. This solution was allowed to cool to 10°C and the crystals were allowed to precipitate at 10°C for 23 hr. The precipitated crystals were collected by filtration, washed with cold water (1 ml) and acetonitrile (5 ml) and vacuum dried at 40°C for 3 hr to give D-tert-leucine·3,4-dimethylbenzenesulfonate (2.54 g). The crystals were analyzed under HPLC analysis condition 1 to find that the D-tert-leucine content was 41.2 wt% and the rate of recovery was 80.0%. Furthermore, an analysis under HPLC analysis condition 2 revealed that the optical purity was 100%ee. Measurement by powder X-ray diffraction method (using CuK_{α} ray as a radiation source) revealed a powder X-ray diffraction pattern as shown in Fig. 2. The melting point was 230-235°C (decomposition).

The obtained salt (1.82 g, containing 0.006 mol of D-tert-leucine) was dissolved in pure water (39 ml) at 45°C, and treated in the same manner as in Example 4 using an ion exchange resin (DIAION WA30, product name, MITSUBISHI CHEMICAL CORPORATION, OH⁻ type, 30 ml) to recover L-tert-leucine. As a result, a transfluent fraction (110 g) was obtained, the D-tert-leucine content was 0.682 wt%, and the rate of recovery of D-tert-leucine was quantitative. Subsequently, a transfluent fraction was concentrated to dryness at 50°C and vacuum dried at 60°C for 4 hr to give D-tert-leucine (0.751 g). The crystals were analyzed under HPLC analysis condition 1 to find that the D-tert-leucine content was 99.4 wt% and the rate of recovery relative to the salt was 99.5%. In addition, an analysis under HPLC analysis condition 2 revealed an optical purity of 100%ee.

### <Example 6>

### Crystallization of D,L-tert-leucine·3,4-dimethylbenzenesulfonate and production of D,L-tert-leucine

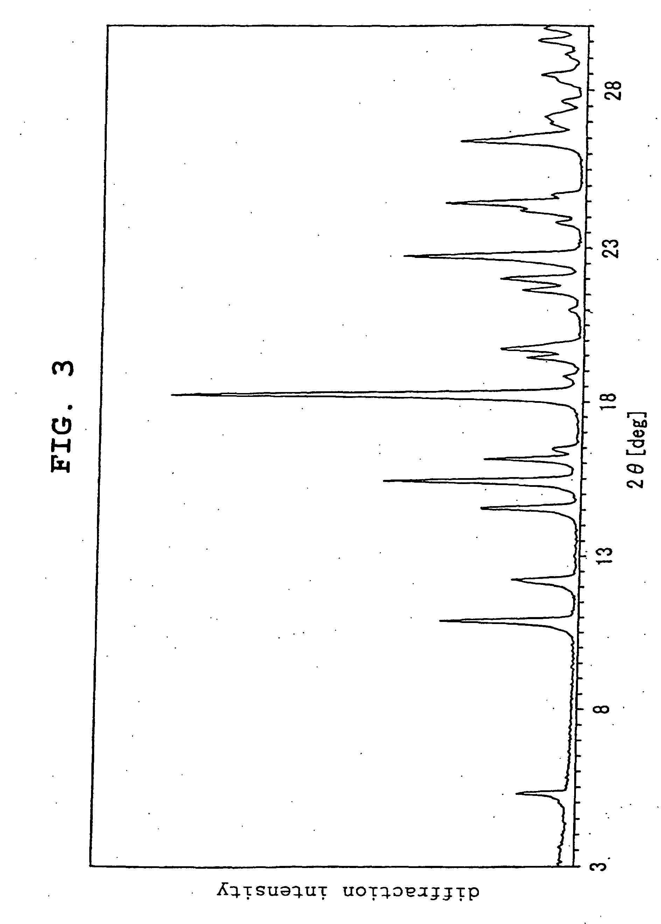

To pure water (10 ml) were added D,L-tert-leucine (1.31 g, 0.010 mol) and the same aqueous 3,4-dimethylbenzenesulfonic acid solution (3.58 g, containing 0.010 mol of 3,4-dimethylbenzenesulfonic acid) as used in Example 1, and the mixture was treated at 80°C to give a homogeneous solution. This solution was allowed to cool to 10°C and the crystals were allowed to precipitate at 10°C for 21 hr. The precipitated crystals were collected by filtration, washed with cold water (1 ml) and acetonitrile (5 ml) and vacuum dried at 40°C for 3 hr to give D,L-tert-leucine·3,4-dimethylbenzenesulfonate (2.42 g). The crystals were analyzed under HPLC analysis condition 1 to find that the D,L-tert-leucine content was 41.3 wt% and the rate of recovery was 76.4%. Measurement by powder X-ray diffraction method (using CuKα ray as a radiation source) revealed a powder X-ray diffraction pattern as shown in Fig. 3. The melting point was 212-218°C (decomposition).

The obtained salt (1.80 g, containing 0.006 mol of D,L-tert-leucine) was dissolved in pure water (50 ml) at 45°C, and treated in the same manner as in Example 4 using an ion exchange resin (DIAION WA30, product name, MITSUBISHI CHEMICAL CORPORATION, OH⁻ type, 30 ml) to recover D,L-tert-leucine. As a result, a transfluent fraction (123 g) was obtained, the D,L-tert-leucine content was 0.606 wt%, and the rate of recovery of D,L-tert-leucine was quantitative. Subsequently, a transfluent fraction was concentrated to dryness at 50°C and vacuum dried at 60°C for 4 hr to give D,L-tert-leucine (0.745 g). The crystals were analyzed under HPLC analysis condition 1 to find that the D,L-tert-leucine content was 99.4 wt% and the rate of recovery relative to the salt was 99.6%.

### <Example 7>

### Crystallization of L-tert-leucine·p-toluenesulfonate

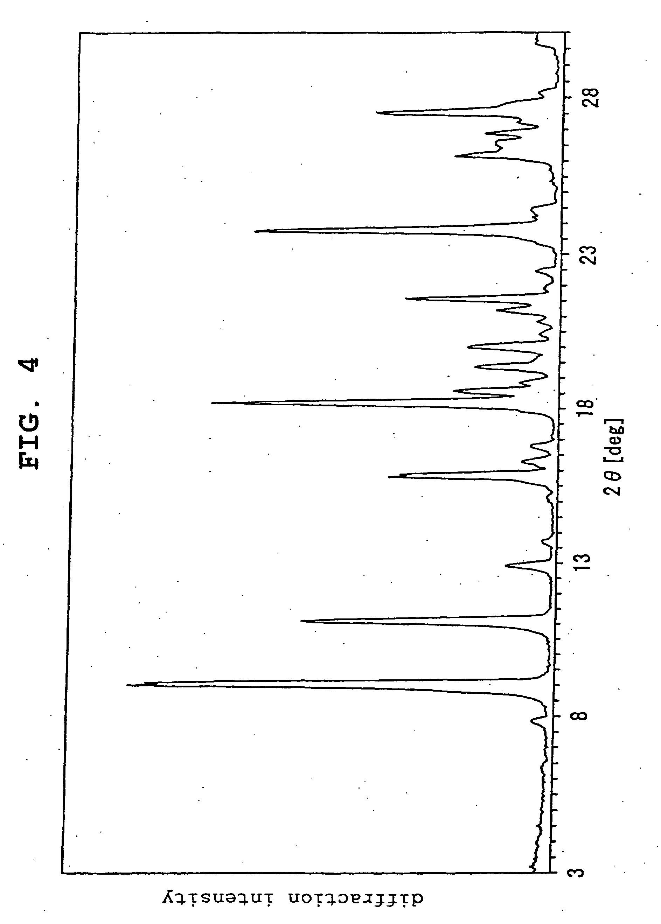

To pure water (18 ml) were added L-tert-leucine (3.94 g, 0.030 mol) and p-toluenesulfonic acid monohydrate (5.71 g, 0.030 mol), and the mixture was treated at 80°C to give a homogeneous solution. This solution was allowed to cool to 10°C and the crystals were allowed to precipitate at 10°C for 24 hr. The precipitated crystals were collected by filtration, washed with cold water (2 ml) and acetonitrile (15 ml) and vacuum dried at 40°C for 3 hr to give L-tert-leucine·p-toluenesulfonate (5.61 g). The crystals were analyzed under HPLC analysis condition 1 to find that the L-tert-leucine content was 43.2 wt% and the rate of recovery was 61.5%. Furthermore an analysis under HPLC analysis condition 2 revealed that the optical purity was 100%ee. Measurement by powder X-ray diffraction method (using CuKα ray as a radiation source) revealed a powder X-ray diffraction pattern as shown in Fig. 4. The melting point was 227-233°C (decomposition).

### <Example 8>

### Crystallization of L-tert-leucine·m-toluenesulfonate

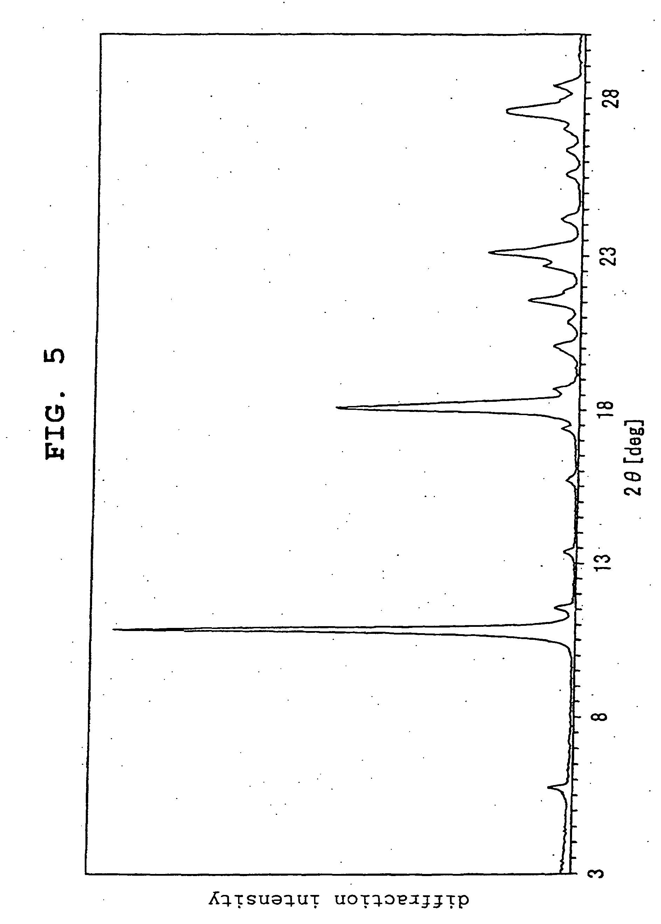

To pure water (15 ml) were added L-tert-leucine (3.94 g, 0.030 mol) and m-toluenesulfonic acid monohydrate (5.71 g, 0.030 mol), and the mixture was treated at 80°C to give a homogeneous solution. This solution was allowed to cool to 10°C and the crystals were allowed to precipitate at 10°C for 20 hr. The precipitated crystals were collected by filtration, washed with cold water (1 ml) and acetonitrile (15 ml) and vacuum dried at 40°C for 5 hr to give L-tert-leucine·m-toluenesulfonate (5.34 g). The crystals were analyzed under HPLC analysis condition 1 to find that the L-tert-leucine content was 43.2 wt% and the rate of recovery was 58.6%. Furthermore, an analysis under HPLC analysis condition 2 revealed that the optical purity was 100%ee. Measurement by powder X-ray diffraction method (using CuKα ray as a radiation source) revealed a powder X-ray diffraction pattern as shown in Fig. 5. The melting point was 217-220°C (decomposition).

### <Example 9>

### Crystallization of L-tert-leucine·p-ethylbenzenesulfonate

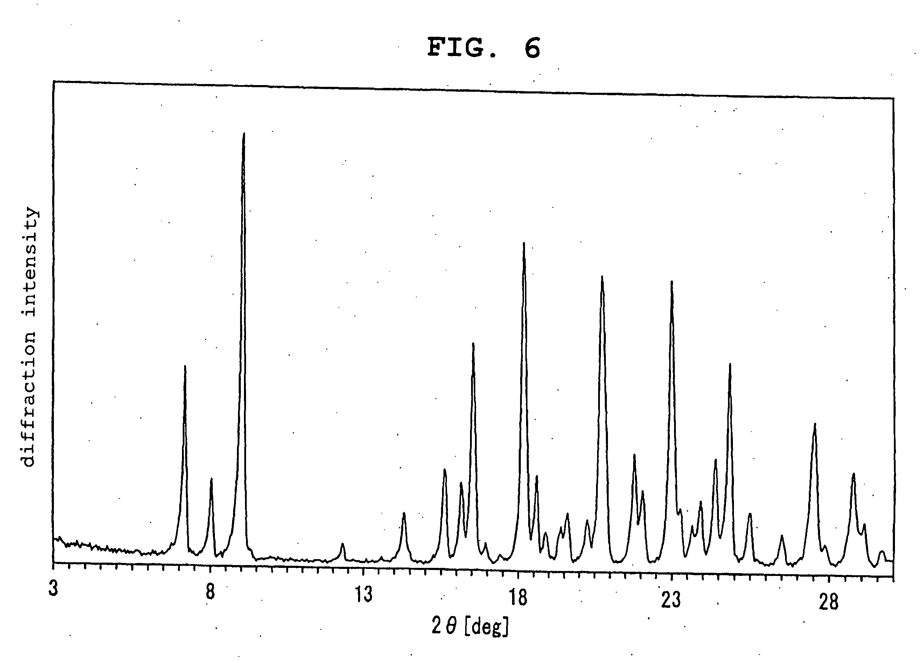

To pure water (15 ml) were added L-tert-leucine (3.94 g, 0.030 mol) and p-ethylbenzenesulfonic acid (5.60 g, 0.030 mol), and the mixture was treated at 80°C to give a homogeneous solution. This solution was allowed to cool to 10°C and the crystals were allowed to precipitate at 10°C for 18 hr. The precipitated crystals were collected by filtration, washed with cold water (2 ml) and acetonitrile (15 ml) and vacuum dried at 40°C for 5 hr to give L-tert-leucine·p-ethylbenzenesulfonate (7.42 g). The crystals were analyzed under HPLC analysis condition 1 to find that the L-tert-leucine content was 41.3 wt% and the rate of recovery was 77.8%. Furthermore, an analysis under HPLC analysis condition 2 revealed that the optical purity was 100%ee. Measurement by powder X-ray diffraction method (using CuKα ray as a radiation source) revealed a powder X-ray diffraction pattern as shown in Fig. 6. The melting point was 221-226°C (decomposition).

### <Example 10>

### Crystallization of L-tert-leucine·p-chlorobenzenesulfonate

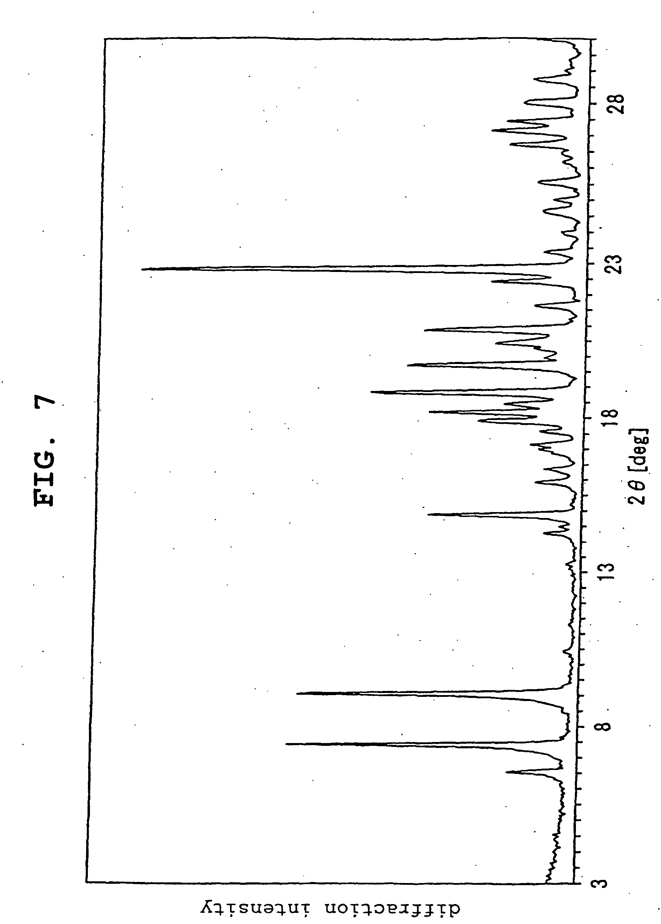

To pure water (12 ml) were added L-tert-leucine (3.94 g, 0.030 mol) and p-chlorobenzenesulfonic acid (5.79 g, 0.030 mol) and the mixture was treated at 80°C to give a homogeneous solution. This solution was allowed to cool to 10°C and the crystals were allowed to precipitate at 10°C for 18 hr. The precipitated crystals were collected by filtration, washed with cold water (2 ml) and acetonitrile (15 ml) and vacuum dried at 40°C for 5 hr to give L-tert-leucine·p-chlorobenzenesulfonate (5.65 g). The crystals were analyzed under HPLC analysis condition 1 to find that the L-tert-leucine content was 39.5 wt% and the rate of recovery was 56.6%. Furthermore, an analysis under HPLC analysis condition 2 revealed that the optical purity was 100%ee. Measurement by powder X-ray diffraction method (using CuKα ray as a radiation source) revealed a powder X-ray diffraction pattern as shown in Fig. 7. The melting point was 214-217°C (decomposition).

### <Example 11>

### Crystallization of L-tert-leucine·3,4-dimethylbenzenesulfonate and production of L-tert-leucine - (3)

To pure water (30 ml) were added L-tert-leucine (3.94 g, 0.030 mol) L-alanine (0.49 g, 0.006 mol), L-aspartic acid (0.12 g, 0.001 mol), L-glutamic acid (0.46 g, 0.003mol) and the same aqueous 3,4-dimethylbenzenesulfonic acid solution (10.78 g, containing 0.030 mol of 3,4-dimethylbenzenesulfonic acid) as used in Example 1, and the mixture was treated at 80°C to give a homogeneous solution. This solution was allowed to cool to 10°C and the crystals were allowed to precipitate at 10°C for 24 hr. The precipitated crystals were collected by filtration, washed with cold water (8 ml) and vacuum dried at 60°C for 3 hr to give L-tert-leucine·3,4-dimethylbenzenesulfonate (7.63 g). The crystals were analyzed under HPLC analysis condition 1 to find that the L-tert-leucine content was 41.3 wt% and the rate of recovery relative to L-tert-leucine used was 80.0%. Furthermore, an analysis under HPLC analysis condition 2 revealed that the optical purity was 100%ee. Furthermore, an analysis by an amino acid analyzer revealed that the L-alanine, L-aspartic acid and L-glutamic acid contents were all not more than 0.1 wt%. Measurement by powder X-ray diffraction method (using CuKα ray as a radiation source) revealed a powder X-ray diffraction pattern similar to that shown in Fig. 1. The melting point was 230-235°C (decomposition).

The obtained salt (4.28 g, containing 0.013 mol of L-tert-leucine) was dissolved in pure water (100 ml) at 70°C, and treated in the same manner as in Example 3 using an ion exchange resin (DIAION WA30, product name, MITSUBISHI CHEMICAL CORPORATION, OH⁻ type, 30 ml) to recover L-tert-leucine. As a result, a transfluent fraction (159 g) was obtained, the L-tert-leucine content was 1.09 wt%, and the rate of recovery of L-tert-leucine relative to the salt was 97.5%. Subsequently, a transfluent fraction was concentrated under reduced pressure to 7.58 g at 50°C and isopropyl alcohol (45 ml) was added. The mixture was heated to 70°C, stirred for 30 min, cooled to 10°C and the crystals were allowed to precipitate for 3 hr. The precipitated crystals were collected by filtration, vacuum dried at 60°C for 4 hr to give L-tert-leucine (1.54 g). The crystals were analyzed under HPLC analysis condition 1 to find that the L-tert-leucine content was 99.3 wt% and the rate of recovery was 88.6%. In addition, an analysis under HPLC analysis condition 2 revealed an optical purity of 100%ee. Furthermore, by an analysis by an amino acid analyzer, L-alanine, L-aspartic acid and L-glutamic acid were not detected.

### <Example 12>

### Crystallization of L-tert-leucine·3,4-dimethylbenzenesulfonate - (3)

To pure water (30 ml) were added L-tert-leucine (3.94 g, 0.030 mol), L-alanine (1.04 g, 0.012 mol) and the same aqueous 3,4-dimethylbenzenesulfonic acid solution (10.78 g, containing 0.030 mol of 3,4-dimethylbenzenesulfonic acid) as used in Example 1, and the mixture was treated at 80°C to give a homogeneous solution. This solution was allowed to cool to 10°C and the crystals were allowed to precipitate at 10°C for 24 hr. The precipitated crystals were collected by filtration, washed with cold water (4 ml) and acetonitrile (15 ml) and vacuum dried at 60°C for 3 hr to give L-tert-leucine·3,4-dimethylbenzenesulfonate (7.42 g). The crystals were analyzed under HPLC analysis condition 1 to find that the L-tert-leucine content was 41.3 wt% and the rate of recovery to L-tert-leucine used was 77.8%. Furthermore, an analysis under HPLC analysis condition 2 revealed that the optical purity was 100%ee. Furthermore, an analysis by an amino acid analyzer did not detect L-alanine. Measurement by powder X-ray diffraction method (using CuKα ray as a radiation source) revealed a powder X-ray diffraction pattern similar to that shown in Fig. 1. The melting point was 230-235°C (decomposition).

### <Example 13>

### Crystallization of L-tert-leucine·3,4-dimethylbenzenesulfonate - (4)

In the same manner as in Example 12 except that L-glutamic acid (0.51 g, 0.003 mol) was added to L-tert-leucine (3.94 g, 0.030 mol) and L-alanine (1.41 g, 0.012 mol), L-tert-leucine·3,4-dimethylbenzenesulfonate (7.08 g) was obtained. The crystals were analyzed under HPLC analysis condition 1 to find that the L-tert-leucine content was 41.2 wt% and the rate of recovery relative to L-tert-leucine used was 74.0%. Furthermore, an analysis under HPLC analysis condition 2 revealed that the optical purity was 100%ee. Furthermore, an analysis by an amino acid analyzer revealed that the L-alanine and L-glutamic acid contents were both not more than 0.1 wt%. Measurement by powder X-ray diffraction method (using CuKα ray as a radiation source) revealed a powder X-ray diffraction pattern similar to that shown in Fig. 1. The melting point was 230-235°C (decomposition).

### Industrial Applicability

According to the present invention, by crystallization of tert-leucine dissolved in a good solvent to tert-leucine such as water and the like as particular substituted benzenesulfonate and dissociation treatment of the salt, tert-leucine can be produced efficiently. Particularly, the method of the present invention can be preferably used for the purpose of selectively separating or purifying tert-leucine from an aqueous solution containing contaminants, such as enzyme reaction solution and the like. In addition, since the substituted benzenesulfonic acid does not need to by purified before use and is economical, the method of the present invention is also an industrially superior method.

This application is based on a patent application No. 61082/2002 filed in Japan, the contents of which are hereby incorporated by reference.

## Claims

1. A method of producing tert-leucine, which comprises reacting tert-leucine with substituted benzenesulfonic acid represented by the following formula (1) 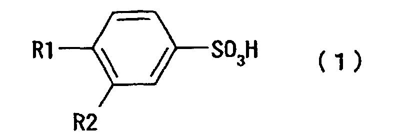 wherein R1 and R2 are each independently a hydrogen atom, an alkyl group or a halogen atom, except a compound wherein R1 and R2 are both hydrogen atoms, in a solvent, to give tert-leucine·substituted benzenesulfonate represented by the following formula (2) 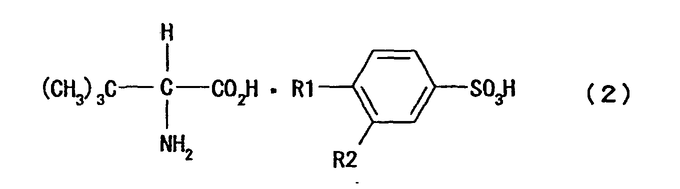 wherein R1 and R2 are the same substituents as above, separating the salt by crystallization and then dissociating the salt.

2. The production method of claim 1, wherein the'solvent is water.

3. The production method of claim 2, wherein the aqueous solvent has pH 0.1-3.0 during crystallization.

4. The production method of any of claims 1 to 3, wherein the tert-leucine is in an optically active form.

5. A tert-leucine·substituted benzenesulfonate represented by the following formula.(2): 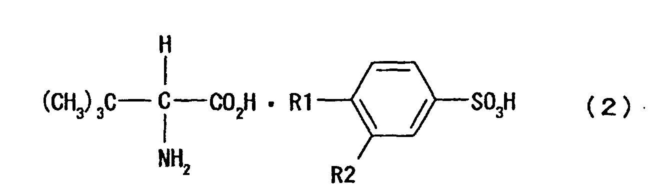 wherein R1 and R2 are each independently a hydrogen atom, an alkyl group or a halogen atom, except a compound wherein R1 and R2 are both hydrogen atoms.
